(19) Europäisches Patentamt
European Patent Office
Office européen des brevets

(11) **EP 1 309 532 B1**

(12) **FASCICULE DE BREVET EUROPEEN**

(45) Date de publication et mention de la délivrance du brevet:
**27.02.2013 Bulletin 2013/09**

(21) Numéro de dépôt: **01963095.3**

(22) Date de dépôt: **10.08.2001**

(51) Int Cl.:
*C07C 37/74* [(2006.01)]     *A23G 1/00* [(2006.01)]
*A23G 1/04* [(2006.01)]     *A23L 3/3472* [(2006.01)]
*A23L 1/30* [(2006.01)]     *C07C 39/00* [(2006.01)]

(86) Numéro de dépôt international:
**PCT/FR2001/002605**

(87) Numéro de publication internationale:
**WO 2002/014251 (21.02.2002 Gazette 2002/08)**

(54) **PROCEDE D'OBTENTION D'EXTRAITS POLYPHENOLIQUES DE FEVES DE CACAO , LES EXTRAITS OBTENUS ET LEURS APPLICATIONS**

**VERFAHREN ZUR HERSTELLUNG VON POLYPHENOLISCHEN EXTRAKTEN AUS KAKAOBOHNEN, AUF DIESE WEISE HERGESTELLTER EXTRAKT UND IHRE VERWENDUNG**

**METHOD FOR OBTAINING COCOA BEAN POLYPHENOL EXTRACTS, RESULTING EXTRACTS AND USES THEREOF**

(84) Etats contractants désignés:
**AT BE CH CY DE DK ES FI FR GB GR IE IT LI LU MC NL PT SE TR**

(30) Priorité: **11.08.2000 FR 0010603**

(43) Date de publication de la demande:
**14.05.2003 Bulletin 2003/20**

(60) Demande divisionnaire:
**07004829.3 / 1 787 970**

(73) Titulaire: **Barry Callebaut AG**
**8005 Zurich (CH)**

(72) Inventeurs:
• **LECOUPEAU, Jean-Paul**
**F-27940 VENABLES (FR)**
• **VERCAUTEREN, Joseph**
**F-34170 CASTELNAU-LE-LEZ (FR)**

(74) Mandataire: **Peaucelle, Chantal et al**
**Cabinet Armengaud Aîné**
**3, Avenue Bugeaud**
**75116 Paris (FR)**

(56) Documents cités:
**EP-A- 0 906 761     EP-A- 0 943 675**
**EP-A- 1 026 164     WO-A-97/36597**

**WO-A-99/45788     GB-A- 2 223 944**
**US-A- 4 352 746     US-A- 6 015 913**

• **PATENT ABSTRACTS OF JAPAN vol. 1998, no. 01, 30 janvier 1998 (1998-01-30) & JP 09 234018 A (MEIJI SEIKA KAISHA LTD), 9 septembre 1997 (1997-09-09)**
• **PATENT ABSTRACTS OF JAPAN vol. 2000, no. 02, 29 février 2000 (2000-02-29) & JP 11 308978 A (MEIJI SEIKA KAISHA LTD), 9 novembre 1999 (1999-11-09)**
• **PATENT ABSTRACTS OF JAPAN vol. 1998, no. 01, 30 janvier 1998 (1998-01-30) & JP 09 224606 A (MEIJI SEIKA KAISHA LTD), 2 septembre 1997 (1997-09-02)**
• **PATENT ABSTRACTS OF JAPAN vol. 1996, no. 02, 29 février 1996 (1996-02-29) & JP 07 274894 A (MEIJI SEIKA KAISHA LTD), 24 octobre 1995 (1995-10-24)**
• **PATENT ABSTRACTS OF JAPAN vol. 1995, no. 11, 26 décembre 1995 (1995-12-26) & JP 07 213251 A (MEIJI SEIKA KAISHA LTD), 15 août 1995 (1995-08-15)**
• **PATENT ABSTRACTS OF JAPAN vol. 018, no. 427 (C-1235), 10 août 1994 (1994-08-10) & JP 06 128164 A (HITOSHI ITO;OTHERS: 01), 10 mai 1994 (1994-05-10)**

Remarques:
Le dossier contient des informations techniques présentées postérieurement au dépôt de la demande et ne figurant pas dans le présent fascicule.

**EP 1 309 532 B1**

**Description**

La présente invention concerne un procédé d'obtention d'extraits à base de composés polyphénoliques à partir de fèves de cacao, les extraits obtenus et leurs applications.

On sait que la graine fraîche de cacao contient environ 40% d'eau, 30 à 35% de lipides, 4 à 6% de polyphénols ou de dérivés de polyphénols, 1,5% de xanthines, le reste étant essentiellement constitué de protéines, d'amidon, de cellulose et de sucres. A cet égard, on peut se reporter notamment aux articles suivants :

-   « Cacao procyanidins : major flavanoids and identification of some minor metabolites » de L.J. Porter, Z. Ma et B.G. Chan, publié dans Phytochemistry vol. 35, n° 5 p1657-1663, 1991 et

-   Epicatechin content in fermented and unfermented cocao beans » de H. Kim et P.G. Keeney, publié dans Journal of Food Science - vol. 49 (1984) p 1090-1092.

On notera que le terme « polyphénol », tel qu'utilisé dans la description et les revendications désigne les polyphénols non substitués, et substitués notamment sous forme de glycosides. Ces polyphénols appartiennent en particulier à la classe des anthocyanines, des flavonoïdes et, des flavanols et leurs oligomères, de types A et/ou B.

De même, on notera que le terme lipide ou matière grasse représente des acides gras libres, des stérols, en particulier des phytostérols, des mono, di et triglycérides. Par pré-traitement, on entend une opération de fermentation et/ou séchage et/ou lavage.

On sait que le cacao est originaire d'Amérique du Sud et que ses fruits ou cabosses sont cueillis, les fèves subissent un pré-traitement qui consiste à fermenter pendant cinq à six jours avant d'être séchées. Au cours de cette fermentation, il se produit un certain nombre de réactions biochimiques se traduisant, notamment, par la destruction de micro-organismes pathogènes, la formation de précurseurs d'arômes et une dégradation partielle des polyphénols par suite d'une oxydation enzymatique ou d'un tannage des protéines. On considère que 70 à 80% des polyphénols sont dégradés lors de la fermentation.

Or, les polyphénols sont les substances naturelles anti-oxydantes et anti-radicalaires les plus puissantes que l'on connaisse. Les extraits polyphénoliques et les préparations qui en contiennent sont classiquement utilisés dans les indications suivantes : troubles circulatoires, insuffisances veino-lymphatiques, fragilité capillaire cutanée, troubles circulatoires de la rétine, crise hémorroïdaire, érythèmes solaires ou liés à l'action de radiations (prévention des dégâts causés par la radiothérapie), hypertension, hypercholestérolémie , diverses affections virales et microbiennes.

Ces dernières années, de nombreuses publications ont révélé au niveau moléculaire les modes d'action par lesquels ils sont capables de lutter contre les affections majeures que sont :

• les maladies cardiovasculaires :

- Antiagrégants plaquettaires (Petroni, A., M. Blasevich, M. Salami, N. Papini, G.F. Montedoro and C. Galli, Inhibition of platelet aggregation and eicosanoid production by phenolic components of olive oil. Thromb Res, 1995. 78(2): p. 151-160)
- Anti-inflammatoires et protecteur de l'oxydation des LDL-cholestérol (Frankel, E., J. Kanner, J. German, E. Parks and J. Kinsella, Inhibition of oxidation of human low-density lipoprotein by phenolic substances in red wine. Lancet, 1993. 341 (8843): p. 454-457)
- Protecteur de l'oxydation des élcasanoïdes (Pace-Asciak, C. R., S. Hahn, E. P. Diamandis, G. Soleas and D. M. Goldberg, The red wine phenolics trans- resveratrol and quercetin block human platelet aggregation and eicosanoid synthesis : implications for protection against coronary heart disease. Clin Chim Acta, 1995.235(2): p. 207-219)
- Anti-athérosclérotiques (Yamakoshi, J., S. Kataoka, T. Koga and T. Ariga, Proanthocyanidin-rich extract from grape seeds attenuates the development of aortic atherosclerosis in cholesterol-fed rabbits. Atherosclerosis, 1999.142(1) : p. 139-149)
- Anti-thrombotiques (Fuhrman, B., A. Lavy and M. Aviram, Consumption of red wine with meals reduces the susceptibility of human plasma and low-densiy lipoprotein to lipid peroxidation. Am J Clin Nutr, 1995.61 (3): p. 549-554)
. l'Alzheimer (Orgogozo, J. M., J. F. Dartigues, S. Lafont, L. Letenneur, D. Commenges, R. Salamon, S. Renaud and M. Breteler, Wine consumption and dementia in the elderly : A prospective community study in the Bordeaux area. Rev Neurol, 1997.153 (3) : p. 185-192)
. le cancer (Jang, M. S., E. N. Cai, G. O. Udeani, K. V. Slowing, C. F. Thomas, C. W. W. Beecher, H. H. S. Fong, N. R. Famsworth, A. D. Kinghom, R. G. Mehta, R. C. Moon and J. M. Pezzuto, Cancer chemopreventive activity of resveratrol, a natural product derived from grapes. Science, 1997.275 (5297) : p. 218-220)

2

EP 0 906 761 décrit un procédé général d'extraction de polyphénols à partir de plantes, dans lequel la matière végétale est dégraissée.

Patent Abstracts of Japan, 1998, et JP 09 234018 se rapportent à une boisson et un produit alimentaire présentant un effet préventif sur les complications de diabètes, obtenu à partir de fèves de cacao dégraissées, de manière à éliminer l'huile qu'elles contiennent.

Patent Abstracts of Japan, 2000, et JP 11 308978 décrivent une boisson et un produit alimentaire pour prévenir l'hypercholestérolémie, comportant un polyphénol de cacao extrait de fèves brutes. Les masses de cacao utilisées sont dégraissées et l'extraction de polyphénol est réalisée en milieu acide.

Patent Abstract of Japan 1998, et JP 09 224 606 concernent une boisson et un produit contenant un composé pour prévenir la carcinogénèse extrait de fèves de cacao dégraissées.

US 4 352 746 se rapporte à un procédé pour l'obtention de substances capables d'inhiber l'oxydation afin de stabiliser des produits alimentaires et cosmétiques. Les enveloppes de fèves de cacao sont citées parmi diverses matières premières. Le procédé comprend le mélange de la matière première concassée ou d'un extrait obtenu avec un solvant léger, avec un véhicule de distillation (triglycérides d'acide gras) et une huile végétale, puis le chauffage et, après plusieurs étapes de traitement, la récupération d'un condensat renfermant les composés recherchés avec le véhicule de co-distillation et une partie de l'huile végétale utilisée.

Patent Abstracts of Japan, 1996, et JP 07 274894 concernent une boisson/produit alimentaire pour prévenir les ulcères qui sont obtenus à partir de fèves de cacao dégraissées.

Patent Abstracts of Japan, 1995, et JP 07 213251 décrivent l'obtention d'une boisson/produit alimentaire contenant un agent oxydant à partir de masses de cacao dégraissées.

US 6 015 913 concerne un procédé de fabrication de beurre de cacao/poudre de cacao à partir de fèves contenant des matières grasses séparées des composés solides.

EP 1 026 164 concerne un procédé d'extraction d'antioxydants polyphénoliques à partir de plantes contenant des purines.

Compte tenu du fait que le cacao contient des polyphénols et de l'importance de l'utilisation des polyphénols dans le domaine médical, on a été amené à tenter d'extraire du cacao, les composés polyphénoliques qu'il contient notamment dans le but de réaliser des aliments et boissons diététiques contenant cet anti-oxydant. Un pré-traitement comportant une fermentation suivie d'une opération de séchage constitue un inconvénient majeur en ce sens qu'il nuit au rendement de l'extraction des polyphénols contenus dans le cacao.

En cherchant à pallier ces inconvénients, les inventeurs ont découvert que l'utilisation de fèves n'ayant pas subi de traitement préalable et la réalisation de l'extraction dans des conditions déterminées permettrait d'obtenir des extraits de composition originale, dotés de propriétés de grand intérêt.

L'invention a donc pour but de fournir un procédé d'extraction de fèves de cacao permettant de disposer d'extraits à forte teneur en polyphénols et enrichis, (par rapport aux teneurs initiales dans les fèves) en certains dérivés lipidiques d'intérêt. Elle vise également à fournir de tels extraits en tant que produits nouveaux.

L'invention vise en outre la mise à profit des propriétés ou des extraits dans diverses applications, notamment dans le domaine alimentaire, cosmétique et thérapeutique.

Le procédé, selon l'invention, d'obtention d'extraits à base de composés polyphénoliques, à partir de fèves de cacao, est caractérisé en ce qu'il comprend

- la mise en oeuvre de fèves fraîches, n'ayant pas subi de pré-traitement, ni de dégraissage, ces fèves étant débarrassées de leurs pulpe et coque, de manière à obtenir des amandes propres,
- le concassage desdites amandes, en présence de solvant,
- la macération des amandes concassées dans des conditions permettant d'extraire les composés recherchés,
- la filtration du mélange de macération,
- la récupération d'extrait renfermant lesdits composés à partir du filtrat.

Les fèves utilisées sont pré-traitées ou non.

Selon une variante de la présente invention, on met en oeuvre des fèves de cacao marchandes, c'est-à-dire des fèves ayant subi un pré-traitement comportant un séchage, dont les grains sont réhumidifiés avant concassage, par exemple avec 30 à 50% d'eau tiède.

L'étape de macération est réalisée à l'aide d'eau ou d'un mélange d'eau et de plusieurs solvants, capables de solubiliser les polyphénols et les lipides, sans altérer leurs propriétés, tels que l'éthanol, l'acétone, le butanol 2, le propanol 2. De préférence, la teneur en solvant est supérieure à 50% en volume.

On opère avantageusement à une température, de l'ordre de 20 à 50°C, pendant 1 heure à plusieurs jours.

Comme le montrent les exemples, une macération de seulement 1 h, avec un solvant tel que l'éthanol 70°C permet d'obtenir des extraits de grande qualité, ce qui présente un grand intérêt pour les applications industrielles du procédé.

Le mélange obtenu est alors filtré et le filtrat traité pour récupérer l'extrait recherché.

De manière avantageuse, le gâteau de filtration est au préalable soumis à une ou plusieurs étapes dé lavage. On utilise en particulier le même solvant que celui mis en oeuvre dans l'étape de macération.

La récupération de l'extrait à base de composés polyphénoliques consiste en particulier en une distillation, conduite de manière à évaporer le solvant et obtenir un extrait.

Les extraits obtenus présentent l'avantage d'une teneur élevée en composés polyphénoliques et un enrichissement, par rapport à la composition des fèves de départ, en phytostérols, notamment en $\beta$-sitostérol.

De tels extraits constituent des produits nouveaux et, à ce titre, entrent également dans le champ de l'invention.

L'invention vise en particulier des extraits caractérisés par une teneur (% en poids par rapport à l'extrait total), en polyphénols de 15 à 65%, en lipides jusqu'à 11% et en xanthines de 5 à 20%.

L'invention vise en particulier les extraits dans lesquels les lipides comportent de 10 à 30% en poids de phytostérols avec de préférence, 7 à 15% de $\beta$-sitostérol. Ce taux préférentiel est atteint lorsque le procédé selon l'invention est mis en oeuvre sur les fèves fraîches.

De tels extraits originaux constituent avantageusement une signature du procédé de l'invention.

On a déjà indiqué en début de description les propriétés mises en évidence chez les polyphénols. De même, les phystostérols constituent des produits de grand intérêt.

Les phytostérols oxydés sont des phytonutriments dont les qualités nutritionnelles en santé publique sont particulièrement bien documentées. De nombreux travaux scientifiques ont montré le rôle, en particulier, du $\beta$-sitostérol dans la protection et la prévention contre certaines maladies.

On peut résumer ainsi ces qualités :

- un effet stimulant sur le système immunitaire en augmentant les défenses immunologiques contre des infections virales et bactériennes (Bouic, P.J.D. et al. International Journal of Immunopharmacology, vol. 18, no. 12, p. 693-700, Dec. 1996),

- un effet hypocholestérolémiant chez l'homme sans changement de diète alimentaire ni de modification de l'activité physique (Métab. Clin. Exper., vol. 38, p. 136-40 (1989) ; American J. Clin. Nutr., vol. 59, p. 1325-31 (1994)),

- combattre les désordres liés au stress (immunosuppression, douleurs et névralgies...), P.J.D. Bouic et al : International Journal of Sports Medicine, 1999,

- combattre les affections de la prostate, Klippel K. F. et al : British Journal of Urology, v. 80 (3), pp. 427-432, Sept. 1997,

- combattre le cancer de la prostate et du sein,

- combattre certaines maladies auto-immune comme le Lupus, le Psoriasis, le Syndrome de fatigue chronique ainsi que la polyarthrite rhumatoïde, (P.J.D. Bouic : Newsletter of the Arthritis Trust of America, Summer 1998),

- maintenir un certain taux de lymphocytes chez des patients atteints du SIDA et donc de prolonger leur vie (Bouic, P.J.D. AIDS Bulletin, v. 6 #3, p 18-20, Sept. 1997), et,

- un effet anti-diabétique, anti-hyperglycémique (M.D. Ivorra et al: Archives of the International Pharmacodyn, v. 296, pp. 224-231, April 1988) ainsi qu'un effet anti-ulcère, anti-inflammatoire et anti-pyrétique (M.B. Gupta et al: Planta medica (Journal of Medicinal Plant Research) vol. 39, p 157-163, 1980).

Les extraits de l'invention, de par leur composition, présentent donc un large spectre d'activité et sont utilisables dans de nombreux domaines d'applications.

On citera, en particulier, l'utilisation des extraits de l'invention dans le domaine alimentaire. Ces extraits constituent en effet des additifs à forte valeur ajoutée.

Ils conviennent, en particulier, à la supplémentation, par exemple, de chocolats, boissons, produits laitiers.

On citera ainsi l'utilisation des extraits selon l'invention comme additifs à certains aliments pour en faire de véritables aliments-santé (aussi appelés aliments fonctionnels ou alicaments) pour lesquels les allégations santé découleraient de l'ensemble des propriétés biologiques qu'on leur connaît.

L'invention vise aussi à protéger les compléments nutritionnels renfermant une quantité efficace d'extraits selon l'invention.

En particulier, les compléments nutritionnels selon l'invention comprennent au moins un extrait selon l'invention à raison de 25 à 300 mg, de préférence de 100 à 200 mg. Leur administration par voie orale, sous forme de comprimés, capsules, gélules est particulièrement adaptée.

EP 1 309 532 B1

Les extraits de l'invention présentent également un intérêt tout particulier dans le domaine cosmétique, où leurs propriétés sont avantageusement mises à profit pour entrer dans la composition de formulations, en tant que principes actifs, ou en combinaison avec d'autres principes actifs.

De telles compositions cosmétiques sont donc caractérisées en ce qu'elles renferment une quantité efficace, pour une application cosmétologique, des extraits de l'invention, en association avec les véhicules classiquement utilisés en cosmétologie. Ces extraits seront ainsi utilisés dans l'élaboration de crèmes, lotions, mousses, savons et autres.

Les propriétés des extraits de l'invention leur confèrent également un grand intérêt en thérapeutique. Comme indiqué plus haut, l'étude des propriétés pharmacologiques de leurs constituants a montré leur efficacité dans diverses affections. Ces propriétés s'accompagnent, en outre, d'une grande innocuité de ces produits, qui présentent donc un indice thérapeutique particulièrement satisfaisant.

L'invention vise ainsi l'utilisation de l'extrait selon l'invention comme source de choix pour l'obtention de principes actifs destinés à une utilisation pharmaceutique.

D'autres caractéristiques et avantages de la présente invention ressortiront de la description faite ci-après en référence aux dessins annexés qui en illustrent deux exemples de mise en oeuvre dépourvus de tout caractère limitatif. Sur les dessins :

- la figure 1 est un schéma représentant les étapes successives du procédé, objet de l'invention, et,
- la figure 2 est un schéma similaire à celui de la figure 1 illustrant la variante du procédé de l'invention selon laquelle on met en oeuvre des fèves marchandes dont les grains sont réhumidifiés avant de subir les opérations de concassage dans un solvant d'extraction et de distillation.

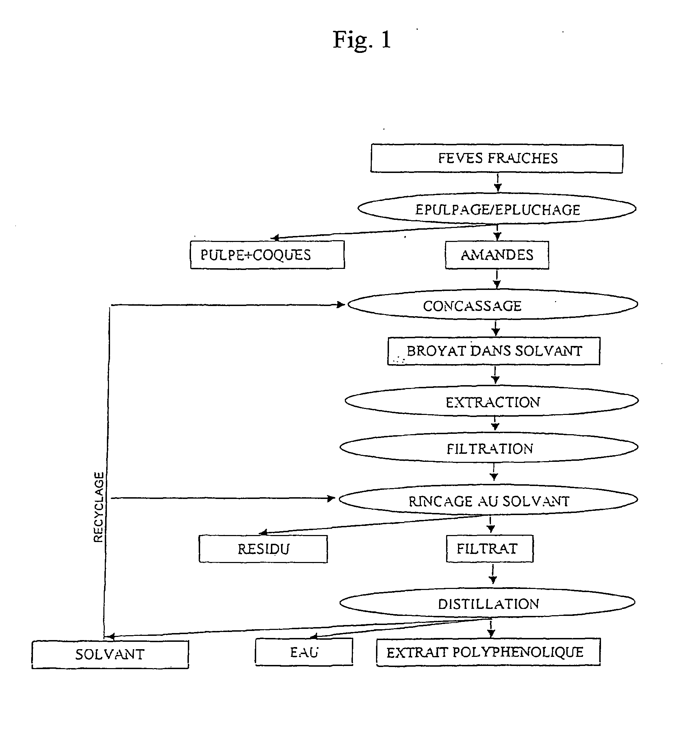

On se réfère en premier lieu à la figure 1.

Selon l'invention, les fèves fraîches, pré-traitées ou non, sont débarrassées de la pulpe et de la coque par une opération d'épulpage/épluchage, par exemple en utilisant un dispositif du type « parmentière ». On obtient ainsi des amandes propres qui sont concassées, par exemple dans un broyeur à couteaux, en présence d'un solvant. Comme précisé ci-dessus, ce solvant est avantageusement choisi dans le groupe comprenant notamment l'eau, l'éthanol, l'acétone, le butanol 2, le propanol 2, en toutes proportions, en mélange avec de l'eau. De préférence, la teneur en solvant est supérieure à 50% en volume (en tenant compte de l'eau apportée par les fèves).

Le mélange amandes concassées/solvant peut être laissé à infuser de quelques heures à plusieurs jours à chaud ou à froid. Si cette infusion est effectuée à chaud, il convient d'éviter les températures trop élevées (c'est-à-dire supérieures à 60°C), afin de limiter l'oxydation chimique et la dégradation chimique des composés à extraire.

Le mélange est ensuite filtré et rincé plusieurs fois à l'aide du solvant utilisé.

On effectue ensuite une distillation en vue d'obtenir un extrait.

Cette distillation est de préférence conduite à une température de 50 à 60°C afin d'éviter une dégradation des composés polyphéniques, sous une pression résiduelle de 12 à 20 KPa, en vue d'évaporer le mélange de solvants contenus dans le filtrat. Comme indiqué sur le schéma de la figure 1, le solvant récupéré lors de l'étape de distillation du filtrat peut être recyclé dans l'étape de concassage des amandes.

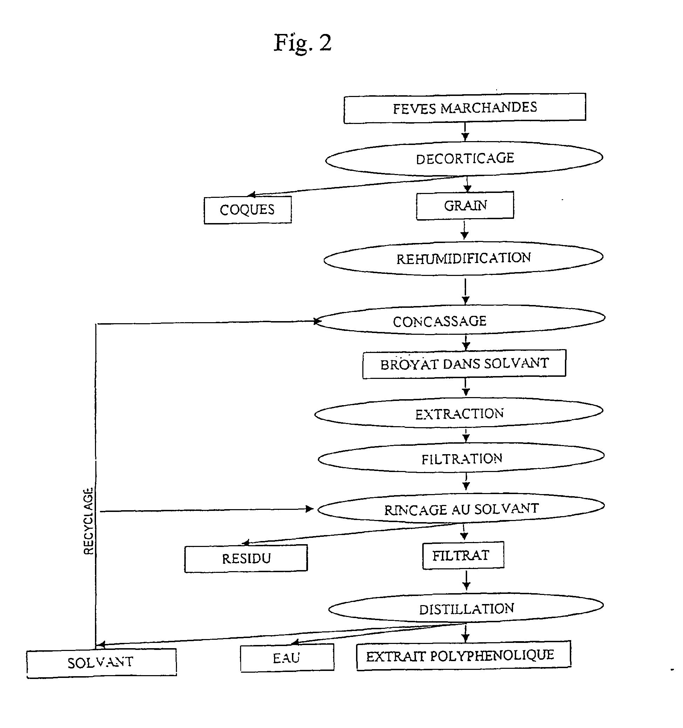

La variante du procédé illustrée par la figure 2 diffère du procédé décrit ci-dessus en référence à la figure 1 uniquement par le fait que le procédé est mis en oeuvre à partir de fèves marchandes de cacao, c'est-à-dire de fèves ayant subi un pré-traitement comportant un séchage, les grains ainsi obtenus étant ensuite soumis à une étape de réhumidification après décorticage, cette étape étant réalisée avec 30 à 50% d'eau tiède, avant l'étape de concassage dans le solvant. Une telle réhumidification permet aux parois cellulaires des amandes de retrouver leur élasticité et donc de ne pas être brisées lors du concassage en présence du solvant. Le pourcentage de lipides extraits est dans ce cas plus important qu'avec les fèves fraîches non réhumidifiées.

On donne ci-après des exemples d'extraits résultants de mises en oeuvre du procédé objet de l'invention.

Dans ces exemples, les xanthines représentent la théobromine et la caféine,

On notera que les pourcentages en polyphénols sont exprimés en équivalent acide gallique, selon la méthode FOLIN CIOCALTEU.

L'activité anti-radicalaire a été évaluée selon le test au DPPH (radical 1,1 diphenyl-2-picryl-hydroxy). Il est nécessaire de connaître la concentration molaire des solutions d'extraits soumis au test. Or, la nature de toutes les molécules présentes n'étant pas connue, une « estimation » d'un poids moléculaire moyen (celui de la catéchine) est alors choisie (arbitrairement) pour exprimer cette molarité. Les résultats sont alors exprimés par le nombre de micromoles nécessaires pour réduire 50% des formes radicalaires du DPPH. D'où, plus la valeur est élevée, moins l'extrait est anti-radicalaire.

Le pourcentage d'extrait/sec (extrait exprimé sur la matière sèche) est déterminé à l'aide de la relation suivante :

5

$$\% \text{ Extrait/sec} = \frac{10\ 000 * E}{W * (100-H)}$$

où :

- E désigne le poids de l'extrait en grammes,
- W désigne le poids des fèves en grammes, et,
- H désigne le taux d'humidité des fèves.

Cette relation permet de rendre comparable les résultats obtenus sur des fèves d'origine différentes. En effet, le taux d'humidité d'une fève est variable selon son origine.

Le dosage des xanthines a été effectué selon la méthode OICCC n°107 (1988).

La composition de la matière grasse a été déterminée d'après la méthode de C.C. Young (voir "The interprétation of GLC Triglycérides Data for the Determination of Cocoa Butter Equivalents in chocolate. A new approach." 1984, JAOCS, 61, p 576-581). L'exploitation des résultats selon PADLEY permet d'interpréter la composition de la partie lipidique (acide gras, stérols, triglycérides...) de l'extrait.

**1) Extraction selon l'invention :**

Variation des rendements d'extraction en fonction du type de solvant (24h de macération)

|  | Eau froide | MeOH (70%) | Eau à 60°C | Acétone (70%) | Propanol 2 (70%) | Ethanol (70%) | Butanol 2 (70%) |
|---|---|---|---|---|---|---|---|
| **% extrait/sec** | 8,8 | 10,6 | 13,4 | 12 | 14,4 | 11,1 | 10,1 |

**Composition extrait (%)**

| Lipides | 0,6 | 0 | 8,7 | 0,1 | 3,6 | 3,5 | 10,4 |
|---|---|---|---|---|---|---|---|
| Xanthines | 8,6 | 6 | 9,4 | 5,6 | 9,9 | 8,5 | 16,1 |
| Polyphénols | 17,2 | 41,2 | 19,1 | 61,1 | 45,5 | 54,3 | 37,6 |

| Activité DPPH | 88,5 | 36,4 | 147,6 | 25,9 | 39 | 31,2 | 41,6 |
|---|---|---|---|---|---|---|---|

L'extrait renfermant la fraction lipidique la plus intéressante associée à la meilleure activité anti-radicalaire (due à la fraction polyphénolique) est obtenue par le mélange de solvant éthanol - eau (70:30 en volume).

Ce solvant est donc choisi de manière préférentielle.

**2) Comparaison sur les extractions de fèves d'origine de Côte d'Ivoire et du Cameroun**

La comparaison est effectuée sur la teneur en stérols de la matière grasse contenue dans les extraits de l'invention.

On rappelle que la composition de la matière grasse de la fève fraîche est peu variable d'une origine à l'autre. Le pourcentage de stérols dans le beurre de cacao est de 0,14 à 0,16%, tandis que le pourcentage de $\beta$- sitostérol est de 0,08 à 0,1%.

Dans le tableau ci-dessous, les extractions sont effectuées sur des fèves du Cameroun avec de l'éthanol à 70%.

|  | 1H | 2H | 4H | 16H | 2j |
|---|---|---|---|---|---|
| % Acides gras | 1,72 | 1,24 | 1,10 | 1,58 | 2,67 |
| % Stérols totaux | 15,59 | 20,44 | 24,80 | 25,16 | 25,60 |
| % β- sitostérol | 9,75 | 12,67 | 14,63 | 14,59 | 14,87 |
| % Diglycérides | 79,91 | 75,65 | 63,12 | 72,20 | 68,73 |
| % Triglycérides | 2,76 | 2,65 | 2,11 | 1,04 | 2,98 |

Dans le tableau ci-dessous, les extractions sont effectuées sur des fèves de Côte d'Ivoire avec de l'éthanol à 70%.

|  | 1H | 2H | 4H | 16H | 2j |
|---|---|---|---|---|---|
| % Acides gras | 11,79 | 15,30 | 14,47 | 18,02 | 17,62 |
| % Stérols totaux | 19,82 | 17,68 | 20,30 | 20,74 | 20,97 |
| % β-sitostérol | 11,54 | 10,51 | 11,95 | 12,18 | 12,18 |
| % Diglycérides | 62,03 | 61,41 | 59,80 | 55,42 | 56,25 |
| % Triglycérides | 3,86 | 2,00 | 1,70 | 0,99 | 1,20 |

On remarquera que le procédé selon l'invention permet d'enrichir la fraction lipidique en stérols et, en particulier, en β-sitostérol, par rapport au beurre de cacao.

**3) Comparaison des extractions selon l'origine des fèves de cacao (24 heures de macération)**

Extraction à l'éthanol 70%.

|  | Cameroun | Guinée EQ | Côte d'Ivoire | Brésil |
|---|---|---|---|---|
| % extrait/sec | 11,1 | 9,5 | 8,4 | 12 |

**Composition extrait %**

| Lipides | 3,5 | 4,9 | 9,2 | 3,4 |
|---|---|---|---|---|
| Xanthines | 8,5 | 8,3 | 10,4 | 14,2 |
| Polyphénols | 54,3 | 40,2 | 47,6 | 38,2 |

| Activité DPPH | 31,2 | 42 | 47,9 | 40,6 |
|---|---|---|---|---|

**4) Extraction sur fèves marchandes (24 heures de macération)**

Extraction à l'éthanol 70%.

| | Côte d'Ivoire |
|---|---|
| **% extrait/sec** | 9,4 |

**Composition extrait (%)**

| Lipides | 9,5 |
|---|---|
| Xanthines | 10,5 |
| Polyphénols | 19 |

| Activité anti-radicalaire | 99 |
|---|---|

**5) Composition dans le domaine agro-alimentaire**

On a indiqué ci-après des exemples non limitatifs d'utilisation d'extraits selon l'invention, obtenus par la mise en oeuvre du procédé défini ci-dessus. Ces extraits peuvent être utilisés en temps que supplémentation dans de nombreux produits alimentaires. Le titulaire a testé l'addition d'extraits obtenus avec de l'éthanol à 70%, dans des produits chocolatés. Dans tous les exemples indiqués ci-après, on a procédé à une comparaison entre le produit avec addition d'extraits et un produit exempt d'une telle addition :

**CHOCOLAT NOIR**

| Composition : pâte de cacao : | 56% |
|---|---|
| Sucre | 26,99% |
| Beurre de cacao | 16% |
| Vanille | 0,01% |
| Extrait de l'invention: | 1% |

Dégustation : le jury était composé de 18 personnes : 12 personnes sur 18 ont préféré le chocolat avec addition d'extrait selon l'invention. Il a été trouvé plus rond et plus aromatique.

**CHOCOLAT AU LAIT**

| Composition : pâte de cacao : | 7,60% |
|---|---|
| Sucre | 41,30% |
| Beurre de cacao | 27,50% |
| Lait gras : | 22,50% |
| Lécithine | 0,59% |
| Vanilline : | 0,01% |
| Extrait de l'invention | 0,50% |

Dégustation : le jury était composé de 15 personnes.
Résultat : couleur légèrement plus rosée; aucune différence significative n'a été relevée en ce qui concerne le goût du produit.

**PREPARATION DE BOISSON POUR DISTRIBUTEUR AUTOMATIQUE**

| Composition sucre : | 52,69% |
|---|---|
| Poudre de cacao fortement dégraissée | 14% |
| Vaniline | 0,01% |
| Lait (0% M.G.) | 33% |
| Extrait de l'invention | 0,30% |

Dégustation : le jury était composé de 9 personnes.
25 grammes de cette préparation ont été additionnés de 200 ml d'eau chaude.
Résultat : aucune différence significative n'a été relevée entre les deux préparations.

Il demeure bien entendu que la présente invention n'est pas limitée aux divers exemples de mise en oeuvre mentionnés ci-dessus mais qu'elle en englobe toutes les variantes.

**6) Composition dans le domaine cosmétique**

Préparation cosmétique anti-solaire pour lutter contre le vieillissement cutané et amincissante.
On réalise une émulsion E/H en mélangeant un filtre solaire avec l'extrait polyphénolique de cacao selon l'invention et des excipients pour crème. Ce sérum associe des propriétés anti-solaire (par la présence d'un filtre solaire et des polyphénols de l'extrait selon l'invention), anti-rides (par la présence des polyphénols de l'extrait selon l'invention) et amincissantes (par la présence des bases xanthiques de l'extrait selon l'invention).

Formulation :

Isopropylméthoxycinnamate et éthyldiisopropylcinnamate (Néo Héliopan E 1000®)  3 %
Extrait de fève de cacao selon l'invention  3 %
Excipients pour sérum E/H  Q.S.

Composition des excipients :

| | |
|---|---|
| - Propylène glycol dicaprylate/dicarate + stearalkonium hectorite + propylène carbonate (Miglyol 840 gel B®) | 20,0 % |
| - Bis-diglycéryl caprylate/caprate/isostéarate/hydroxystéarate adipate (softisan 649®) | 5,0% |
| - Isostéaryl diglycéryl succinate (Imwitor 780 K®) | 5,0 % |
| - Huile de paraffine | 8,0 % |
| - Paraffine solide | 3,0 % |
| - Sulfate de magnésium | 2,0 % |
| - Eau | q.s. 100, 0 % |

**Revendications**

1. Procédé d'obtention d'extraits à base de composés polyphénoliques contenus dans le cacao, **caractérisé en ce qu'**il comprend :

   - la mise en oeuvre de fèves fraîches, n'ayant pas subi de pré-traitement, ni de dégraissage, ces fèves étant débarrassées de leurs pulpe et coque, de manière à obtenir des amandes propres,
   - le concassage desdites amandes, en présence de solvant(s),
   - la macération des amandes concassées réalisée dans de l'eau ou dans un mélange d'eau et de solvant, le solvant étant choisi dans le groupe comprenant l'éthanol, l'acétone, le butanol 2, et le propanol 2,
   - la filtration du mélange de macération,
   - la récupération d'un extrait renfermant lesdits composés à partir du filtrat.

2. Procédé d'obtention d'extraits à base de composés polyphénoliques contenus dans le cacao, **caractérisé en ce qu'**il comprend :

   - la mise en oeuvre de fèves fraîches, ayant subi un pré-traitement consistant en un séchage et optionnellement un lavage, n'ayant pas subi de dégraissage, ces fèves étant débarrassées de leurs pulpe et coque, de manière à obtenir des amandes propres,
   - le concassage desdites amandes, en présence de solvant(s),
   - la macération des amandes concassées, réalisée dans de l'eau ou dans un mélange d'eau et de solvant, le solvant étant choisi dans le groupe comprenant l'éthanol, l'acétone, le butanol 2, et le propanol 2,
   - la filtration du mélange de macération,
   - la récupération d'un extrait renfermant lesdits composés à partir du filtrat.

3. Procédé selon la revendication 1 ou 2, **caractérisé en ce qu'**on utilise un mélange solvant/eau avec une teneur en solvant supérieure à 50% en volume.

4. Procédé selon l'une quelconque des revendications 1 à 3, **caractérisé en ce que** l'étape de macération est réalisée durant 1 h à plusieurs jours, à chaud ou à froid.

5. Procédé selon l'une quelconque des revendications 1 à 4, **caractérisé en ce que** la récupération de l'extrait est réalisée par distillation du filtrat obtenu, à une température comprise entre 50 et 60 °C, sous une pression résiduelle de 12 à 20 KPa.

6. Extraits à base de composés polyphénoliques contenus dans le cacao susceptibles d'être obtenus par le procédé selon l'une quelconque des revendications 1 à 5.

7. Extraits à base de composés polyphénoliques, **caractérisés par** une teneur (% en poids par rapport à l'extrait total), en polyphénols de 15 à 65%, en lipides jusqu'à 11% et en xanthines de 5 à 20%, lesdits lipides comportant de 10 à 30% en poids de phystostérols avec de préférence, 7 à 15% de β-sitostérol.

8. Utilisation des extraits selon la revendication 6 ou 7, dans le domaine alimentaire, en tant qu'additifs, notamment pour la supplémentation de chocolats, boissons, produits laitiers.

**9.** Utilisation des extraits selon la revendication 6 ou 7, dans le domaine alimentaire, pour la fabrication d'aliments fonctionnels.

**10.** Compléments nutritionnels **caractérisés en ce qu'**ils renferment une quantité efficace d'extraits selon la revendication 6 ou 7.

**11.** Compléments nutritionnels selon la revendication 10, **caractérisés en ce qu'**ils comprennent au moins un extrait selon la revendication 6 ou 7, à raison de 25 à 300 mg, de préférence de 100 à 200 mg.

**12.** Compléments nutritionnels selon la revendication 10 ou 11, **caractérisés en ce qu'**ils sont administrables par voie orale, en particulier sous forme de comprimés, capsules, gélules.

**13.** Utilisation des extraits selon la revendication 6 ou 7, dans le domaine cosmétique, en tant que principes actifs ou en combinaison avec d'autres principes actifs.

**14.** Compositions cosmétiques, **caractérisées en ce qu'**elles renferment une quantité efficace, pour une application cosmétique, des extraits selon la revendication 6 ou 7, en association avec les véhicules classiquement utilisés en cosmétologie.

**15.** Compositions cosmétiques selon la revendication 14, **caractérisées en ce qu'**elles se présentent sous forme de crèmes, lotions, mousses, savons.

**16.** Utilisation de l'extrait selon la revendication 6 ou 7, comme source pour l'obtention de principes actifs dans le domaine pharmaceutique.

**Claims**

**1.** A process for obtaining extracts based on polyphenol compounds contained in cocoa, comprising:

- using fresh beans, not having undergone a pre-treatment, or defatting, said beans having their pulp and shell removed, in such a way as to obtain clean nibs,
- grinding said nibs, in the presence of solvent(s),
- macerating the ground nibs in water or a mixture of water and solvent, the solvent being chosen from the group comprising ethanol, acetone, 2-butanol, and 2-propanol,
- filtrating the maceration mixture,
- recovering an extract containing said compounds from the filtrate.

**2.** A process for obtaining extracts based on polyphenol compounds contained in cocoa, comprising

- using fresh beans, having undergone a pre-treatment consisting in a drying and optionally a washing, having undergone no defatting, said beans having their pulp and shell removed, in such a way as to obtain clean kernels,
- grinding said kernels, in the presence of solvent(s),
- macerating the ground kernels, in water or a mixture of water and solvent,
- filtrating the maceration mixture,
- recovering an extract containing said compounds from the filtrate.

**3.** The process according to claim 1 or 2, **characterised in that** a solvent/water mixture is used with a solvent content greater than 50% by volume.

**4.** The process according to any one of claims 1 to 3, **characterised in that** the maceration step is carried out over 1 hour to several days, under hot or cold conditions.

**5.** The process according to any one of claims 1 to 4, **characterised in that** the recovery of the extract is carried out by distillation of the obtained filtrate, at a temperature comprised between 50 and 60°C, under a residual pressure of 12 to 20 Kpa.

6. Extracts based on polyphenol compounds contained in cocoa susceptible to be obtained by the process according to anyone of claims 1 to 5.

7. Extracts based on polyphenol compounds, **characterised by** a content (% by weight relative to the total extract) of 15 to 65% polyphenols, up to 11 % lipids and 5 to 20% xanthines, said lipids comprising 10 to 30% by weight of phystosterols with preferably 7 to 15% ß-sitosterol.

8. Use of the extracts according to claim 6 or 7, in the field of foodstuffs, as additives, in particular to supplement chocolates, drinks and dairy products.

9. Use of the extracts according to claim 6 or 7, in the field of foodstuffs, for the manufacture of functional foods.

10. Nutritional supplements **characterised in that** they contain an effective amount of extracts according to claim 6 or 7.

11. Nutritional supplements according to claim 10, **characterised in that** they comprise at least an extract according to claim 6 or 7, in an amount of 25 to 300 mg, preferably 100 to 200 mg.

12. Nutritional supplements according to claim 10 or 11, **characterised in that** they can be administered by oral route, in particular in the form of tablets, capsules or gelatin capsules.

13. Use of the extracts according to claim 6 or 7, in the cosmetic field, as active ingredients or in combination with other active ingredients.

14. Cosmetic compositions, **characterised in that** they contain an effective quantity, for a cosmetological application, of extracts according to claim 6 or 7, in combination with the vehicles usually used in cosmetology.

15. Cosmetic compositions according to claim 14, **characterised in that** they are presented in the form of creams, lotions, mousses, soaps.

16. Use of the extract according to claim 6 or 7, as a source for obtaining active ingredients in the pharmaceutical field.

**Patentansprüche**

1. Verfahren zur Gewinnung von Extrakten auf der Basis von in Kakao enthaltenen Polyphenolverbindungen, **dadurch gekennzeichnet, dass** es folgende Schritte umfasst:

   - Bereitstellen von frischen Kakaobohnen, die keine Vorbehandlung und auch keine Entfettung erfahren haben, wobei diese Kakaobohnen von ihrer Pulpe und Schale befreit wurden, so dass man saubere Kerne erhält;
   - Zerkleinern der Kerne in Gegenwart eines oder mehrerer Lösungsmittel;
   - Mazerieren der zerkleinerten Kerne in Wasser oder in einem Gemisch aus Wasser und Lösungsmittel, wobei das Lösungsmittel aus der Gruppe ausgewählt ist, die aus Ethanol, Aceton, 2-Butanol und 2-Propanol besteht;
   - Filtrieren des mazerierten Gemischs;
   - Gewinnen eines Extrakts, der die obigen Verbindungen enthält, aus dem Filtrat.

2. Verfahren zur Gewinnung von Extrakten auf der Basis von in Kakao enthaltenen Polyphenolverbindungen, **dadurch gekennzeichnet, dass** es folgende Schritte umfasst:

   - Bereitstellen von frischen Kakaobohnen, die eine Vorbehandlung erfahren haben, die aus Trocknen und gegebenenfalls Waschen bestand, und die keine Entfettung erfahren haben, wobei diese Kakaobohnen von ihrer Pulpe und Schale befreit wurden, so dass man saubere Kerne erhält;
   - Zerkleinern der Kerne in Gegenwart eines oder mehrerer Lösungsmittel;
   - Mazerieren der zerkleinerten Kerne in Wasser oder in einem Gemisch aus Wasser und Lösungsmittel, wobei das Lösungsmittel aus der Gruppe ausgewählt ist, die aus Ethanol, Aceton, 2-Butanol und 2-Propanol besteht;
   - Filtrieren des mazerierten Gemischs;
   - Gewinnen eines Extrakts, der die obigen Verbindungen enthält, aus dem Filtrat.

3. Verfahren gemäß Anspruch 1 oder 2, **dadurch gekennzeichnet, dass** ein Lösungsmittel/Wasser-Gemisch mit einem Lösungsmittelgehalt von über 50 Vol.-% verwendet wird.

4. Verfahren gemäß einem der Ansprüche 1 bis 3, **dadurch gekennzeichnet, dass** der Schritt des Mazerierens während 1 h bis mehreren Tagen heiß oder kalt durchgeführt wird.

5. Verfahren gemäß einem der Ansprüche 1 bis 4, **dadurch gekennzeichnet, dass** die Gewinnung des Extrakts durch Destillation des erhaltenen Filtrats bei einer Temperatur im Bereich von 50 bis 60 °C unter einem Restdruck von 12 bis 20 kPa durchgeführt wird.

6. Extrakte auf der Basis von in Kakao enthaltenen Polyphenolverbindungen, die nach dem Verfahren gemäß einem der Ansprüche 1 bis 5 erhältlich sind.

7. Extrakte auf der Basis von Polyphenolverbindungen, **gekennzeichnet durch** einen Gehalt (Gew.-%, bezogen auf den Gesamtextrakt) an Polyphenolen von 15 bis 65%, an Lipiden von bis zu 11% und an Xanthinen von 5 bis 20%, wobei die Lipide 10 bis 30 Gew.-% Phytosterole mit vorzugsweise 7 bis 15% β-Sitosterol umfassen.

8. Verwendung der Extrakte gemäß Anspruch 6 oder 7 im Lebensmittelbereich als Zusatzstoffe, insbesondere zur Ergänzung von Schokoladen, Getränken, Milchprodukten.

9. Verwendung der Extrakte gemäß Anspruch 6 oder 7 im Lebensmittelbereich zur Herstellung von funktionellen Lebensmitteln.

10. Nahrungsergänzungsmittel, **dadurch gekennzeichnet, dass** sie eine wirksame Menge von Extrakten gemäß Anspruch 6 oder 7 umfassen.

11. Nahrungsergänzungsmittel gemäß Anspruch 10, **dadurch gekennzeichnet, dass** sie wenigstens einen Extrakten gemäß Anspruch 6 oder 7 in einer Menge von 25 bis 300 mg, vorzugsweise 100 bis 200 mg, umfassen.

12. Nahrungsergänzungsmittel gemäß Anspruch 10 oder 11, **dadurch gekennzeichnet, dass** sie oral, insbesondere in Form von Tabletten, Kapseln oder Gelatinekapseln, verabreichbar sind.

13. Verwendung der Extrakte gemäß Anspruch 6 oder 7 im Kosmetikbereich als Wirkstoffe oder in Kombination mit anderen Wirkstoffen.

14. Kosmetische Zusammensetzungen, **dadurch gekennzeichnet, dass** sie eine für eine kosmetische Anwendung wirksame Menge der Extrakte gemäß Anspruch 6 oder 7 in Verbindung mit in der Kosmetik herkömmlicherweise verwendeten Trägern umfassen.

15. Kosmetische Zusammensetzungen gemäß Anspruch 14, **dadurch gekennzeichnet, dass** sie in Form von Cremes, Lotionen, Schäumen oder Seifen vorliegen.

16. Verwendung des Extrakts gemäß Anspruch 6 oder 7 als Quelle für die Gewinnung von Wirkstoffen im pharmazeutischen Bereich.

# Fig. 1

FEVES FRAICHES

EPULPAGE/EPLUCHAGE

PULPE+COQUES

AMANDES

CONCASSAGE

BROYAT DANS SOLVANT

EXTRACTION

FILTRATION

RINCAGE AU SOLVANT

RECYCLAGE

RESIDU

FILTRAT

DISTILLATION

SOLVANT

EAU

EXTRAIT POLYPHENOLIQUE

# Fig. 2

```
                        ┌──────────────────────────┐
                        │     FEVES MARCHANDES      │
                        └──────────────────────────┘
                                     │
                                     ▼
                        ╭──────────────────────────╮
                        │       DECORTICAGE         │
                        ╰──────────────────────────╯
              ┌─────────┐                    │
              │ COQUES  │◄───────────  ┌──────────┐
              └─────────┘              │  GRAIN   │
                                       └──────────┘
                                            │
                                            ▼
                        ╭──────────────────────────╮
                        │     REHUMIDIFICATION      │
                        ╰──────────────────────────╯
                                            │
                                            ▼
                        ╭──────────────────────────╮
              ┌────────►│       CONCASSAGE          │
              │         ╰──────────────────────────╯
              │                     │
              │         ┌──────────────────────────┐
              │         │   BROYAT DANS SOLVANT     │
              │         └──────────────────────────┘
              │                     │
              │                     ▼
              │         ╭──────────────────────────╮
              │         │        EXTRACTION         │
              │         ╰──────────────────────────╯
              │                     │
              │                     ▼
              │         ╭──────────────────────────╮
              │         │        FILTRATION         │
              │         ╰──────────────────────────╯
              │                     │
   RECYCLAGE  │                     ▼
              │         ╭──────────────────────────╮
              └────────►│    RINCAGE AU SOLVANT     │
              │         ╰──────────────────────────╯
              │              │              │
              │      ┌──────────┐    ┌──────────┐
              │      │  RESIDU  │    │  FILTRAT │
              │      └──────────┘    └──────────┘
              │                            │
              │                            ▼
              │         ╭──────────────────────────╮
              │         │       DISTILLATION        │
              │         ╰──────────────────────────╯
              │              │      │      │
    ┌──────────┐   ┌──────────┐  ┌────────────────────────┐
    │ SOLVANT  │   │   EAU    │  │  EXTRAIT POLYPHENOLIQUE │
    └──────────┘   └──────────┘  └────────────────────────┘
```

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0906761 A
- JP 9234018 A
- JP 11308978 A
- JP 09224606 B
- US 4352746 A
- JP 7274894 A
- JP 7213251 A
- US 6015913 A
- EP 1026164 A

**Littérature non-brevet citée dans la description**

- **L.J. PORTER ; Z. MA ; B.G. CHAN.** Cacao procyanidins : major flavanoids and identification of some minor metabolites. *Phytochemistry,* 1991, vol. 35 (5), 1657-1663
- **H. KIM ; P.G. KEENEY.** Epicatechin content in fermented and unfermented cocao beans. *Journal of Food Science,* 1984, vol. 49, 1090-1092
- **PETRONI, A. ; M. BLASEVICH ; M. SALAMI ; N. PAPINI ; G.F. MONTEDORO ; C. GALLI.** Inhibition of platelet aggregation and eicosanoid production by phenolic components of olive oil. *Thromb Res,* 1995, vol. 78 (2), 151-160
- **FRANKEL, E. ; J. KANNER ; J. GERMAN ; E. PARKS ; J. KINSELLA.** Inhibition of oxidation of human low-density lipoprotein by phenolic substances in red wine. *Lancet,* 1993, vol. 341 (8843), 454-457
- **PACE-ASCIAK, C. R. ; S. HAHN ; E. P. DIAMANDIS ; G. SOLEAS ; D. M. GOLDBERG.** The red wine phenolics trans- resveratrol and quercetin block human platelet aggregation and eicosanoid synthesis : implications for protection against coronary heart disease. *Clin Chim Acta,* 1995, vol. 235 (2), 207-219
- **YAMAKOSHI, J. ; S. KATAOKA ; T. KOGA ; T. ARIGA.** Proanthocyanidin-rich extract from grape seeds attenuates the development of aortic atherosclerosis in cholesterol-fed rabbits. *Atherosclerosis,* 1999, vol. 142 (1), 139-149
- **FUHRMAN, B. ; A. LAVY ; M. AVIRAM.** Consumption of red wine with meals reduces the susceptibility of human plasma and low-densiy lipoprotein to lipid peroxidation. *Am J Clin Nutr,* 1995, vol. 61 (3), 549-554
- **ORGOGOZO, J. M. ; J. F. DARTIGUES ; S. LAFONT ; L. LETENNEUR ; D. COMMENGES ; R. SALAMON ; S. RENAUD ; M. BRETELER.** Wine consumption and dementia in the elderly : A prospective community study in the Bordeaux area. *Rev Neurol,* 1997, vol. 153 (3), 185-192
- **JANG, M. S. ; E. N. CAI, G. O. UDEANI ; K. V. SLOWING ; C. F. THOMAS ; C. W. W. BEECHER ; H. H. S. FONG ; N. R. FAMSWORTH ; A. D. KINGHOM ; R. G. MEHTA ; R. C. MOON.** Cancer chemopreventive activity of resveratrol, a natural product derived from grapes. *Science,* 1997, vol. 275 (5297), 218-220
- **BOUIC, P.J.D. et al.** *International Journal of Immunopharmacology,* 1996, vol. 18 (12), 693-700
- *Métab. Clin. Exper.,* vol. 38, 136-40
- *American J. Clin. Nutr.,* 1994, vol. 59, 1325-31
- **KLIPPEL K. F. et al** *British Journal of Urology,* Septembre 1997, vol. 80 (3), 427-432
- **P.J.D. BOUIC.** *Newsletter of the Arthritis Trust of America,* 1998
- **BOUIC, P.J.D.** AIDS Bulletin. Septembre 1997, vol. 6 #3, 18-20
- **M.D. IVORRA et al.** Archives of the International Pharmacodyn. Avril 1988, vol. 296, 224-231
- **M.B. GUPTA et al.** *Planta medica,* 1980, vol. 39, 157-163
- The interprétation of GLC Triglycérides Data for the Determination of Cocoa Butter Equivalents in chocolate. A new approach. *JAOCS,* 1984, vol. 61, 576-581